Europäisches Patentamt

(19) European Patent Office

Office européen des brevets

(11) Numéro de publication: **0 147 313**
**A2**

## (12) DEMANDE DE BREVET EUROPEEN

(21) Numéro de dépôt: **84402662.5**

(22) Date de dépôt: **19.12.84**

(51) Int. Cl.⁴: **A 61 F 9/06**
**A 62 B 18/04, A 42 B 3/00**

(30) Priorité: **27.12.83 FR 8320849**

(43) Date de publication de la demande:
**03.07.85 Bulletin 85/27**

(84) Etats contractants désignés:
**BE CH DE GB IT LI LU NL SE**

(71) Demandeur: **COMMISSARIAT A L'ENERGIE ATOMIQUE Etablissement de Caractère Scientifique Technique et Industriel**
**31/33, rue de la Fédération**
**F-75015 Paris(FR)**

(72) Inventeur: **Guillaumot, Jacques**
**50, route Nationale 113**
**F-34690 Fabrègue(FR)**

(74) Mandataire: **Mongrédien, André et al,**
**c/o BREVATOME 25, rue de Ponthieu**
**F-75008 Paris(FR)**

(54) Heaume ergonomique.

(57) Le heaume selon l'invention comporte une pompe (8) équipée d'un système de commande (9) qui assure simultanément l'envoi d'air sur la partie en forme de casque (2) et la manoeuvre de la visière (4); ce système de commande a une position "marche" assurant l'abaissement de la visière (4), son maintien en position abaissée et la ventilation normale et une position "repos" assurant le relevage de la visière (4), son maintien en position relevée et une ventilation réduite formant écran autour du visage de l'opérateur.

Application à la protection de personnel travaillant en atmosphère poussiéreuse.

FIG. 1

Croydon Printing Company Ltd

## HEAUME ERGONOMIQUE

La présente invention a pour objet un heaume ergonomique, c'est-à-dire un casque de protection, léger, pratique et agréable à porter par l'utilisateur.

Lorsqu'on effectue certains travaux qui dégagent une grande quantité de poussières ou d'étincelles comme les travaux de soudage, meulage, sablage ou pulvérisation, il est indispensable que l'opérateur soit protégé, surtout au niveau du visage. Il en est de même pour les travaux de peinture et dans le domaine de l'agriculture pour la pulvérisation de pesticides. Il existe actuellement un certain nombre de dispositifs assurant cette protection. Ces dispositifs peuvent être des heaumes escamotables en polypropylène ou en PVC avec une fenêtre en mica pour les soudeurs et les meuleurs mais qui n'ont pas de ventilation et sont donc assez désagréables à porter, car ils tiennent chaud. Il existe aussi des masques ou demi-masques éventuellement munis de cartouches filtrantes, des masques filtrants à protection oculaire, ainsi que des masques munis d'un système autonome d'adduction d'air, celui-ci étant fourni par une pompe extérieure ou par une bouteille d'air comprimé. Enfin, il existe des tenues ventilées complètes et étanches couvrant la tête, le buste ou tout le corps.

Tous ces équipements présentent l'inconvénient de ne pas assurer simultanément la protection contre les nuisances (poussières, vapeurs ou étincelles) et le confort ergonomique de l'opérateur : ceci entraîne soit le port d'équipements sommaires à protection faible mais assurant un certain confort soit l'usage d'équipements assurant une protection efficace mais qui entraînent une fatigue rapide, ce qui fait qu'au bout d'un certain temps les opérateurs ne les

B 8069.3 JR

portent plus. D'autre part, ces équipements assurant une protection efficace gênent le travail de l'utilisateur et, de plus, ils sont onéreux, ce qui explique leur diffusion restreinte.

La présente invention a justement pour objet de remédier à ces inconvénients en proposant un heaume ergonomique léger et agréable à porter qui assure simultanément une bonne protection contre les nuisances et un bon confort de l'opérateur.

Le heaume objet de l'invention comporte, de manière connue, une partie en forme de casque équipée d'une visière déplaçable entre une position abaissée et une position relevée, ainsi qu'un système d'adduction d'air à cette partie en forme de casque au moyen d'une source d'air extérieure ; selon l'invention, ce heaume comporte un moyen de commande assurant simultanément la ventilation et la manoeuvre de la visière, ledit moyen de commande ayant une position "marche" assurant simultanément l'abaissement de la visière et son maintien en position abaissée ainsi que la ventilation normale et une position "repos" assurant simultanément le relevage de la visière et son maintien en position relevée ainsi qu'une arrivée d'air réduite formant écran autour du visage de l'opérateur.

Selon une autre caractéristique de l'invention, lesdits moyens de commande comprennent :
- une pompe servant de source d'air extérieure, cette pompe étant équipée d'une commande ayant une position "marche" assurant la ventilation normale et une position "repos" assurant une arrivée d'air réduite, cette commande assurant en outre la manoeuvre de la visière,
- un système de filtration des polluants équipant ladite pompe,
- une conduite souple reliant cette pompe à ladite partie en forme de casque, et

B 8069.3 JR

- plusieurs tuyaux souples reliant l'extrémité de cette conduite souple opposée à la pompe à différents orifices de sortie.

Dans un mode préféré de réalisation, l'un au moins desdits tuyaux souples débouche à une extrémité d'un tube présentant une fente longitudinale dont la largeur augmente au fur et à mesure qu'on s'éloigne de l'extrémité où débouche ledit tuyau souple, assurant ainsi un flux d'air laminaire.

Dans un premier mode de réalisation, l'un aux moins desdits tuyaux souples débouche dans un cylindre contenant un piston mobile relié à une extrémité d'un ressort en spirale coopérant avec ladite visière.

Dans un deuxième mode de réalisation, le heaume ergonomique objet de l'invention comporte :
- une source de courant électrique, et
- un élément en matériau à mémorisation thermique inverse, ledit élément étant relié électriquement à la source de courant et coopérant avec ladite visière.

Dans ce cas, ledit élément en matériau à mémorisation thermique inverse est de préférence un ressort en spirale dont une extrémité est fixée à la partie en forme de casque et dont l'autre extrémité est fixée à un axe mobile en rotation par rapport à la partie en forme de casque et solidaire de la visière au cours de cette rotation.

Selon un autre aspect intéressant de l'invention, la partie en forme de casque du heaume est tapissée intérieurement d'une couche de liège et un micro-émetteur-récepteur de radio peut être incorporé dans la partie en forme de casque.

L'invention apparaîtra mieux à la lecture de la description qui va suivre, donnée à titre d'exemple purement illustratif et nullement limitatif, en référence aux dessins annexés, dans lesquels :

B 8069.3 JR

4                           **0147313**

- la figure 1 est une vue schématique en perspective de l'ensemble du heaume ergonomique objet de l'invention,

- la figure 2 est une vue en coupe de la partie en forme de casque de ce heaume,

- la figure 3 est une vue schématique en perspective de l'un des tubes équipant la partie en forme de casque afin d'assurer un flux laminaire,

- la figure 4 est une vue schématique d'un premier mode de réalisation du système automatique de relevage et d'abaissement de la visière, et

- la figure 5 est une vue semblable à la figure 4 illustrant un deuxième mode de réalisation du système automatique de relevage et d'abaissement automatique de la visière à l'aide d'un matériau à mémorisation thermique inverse.

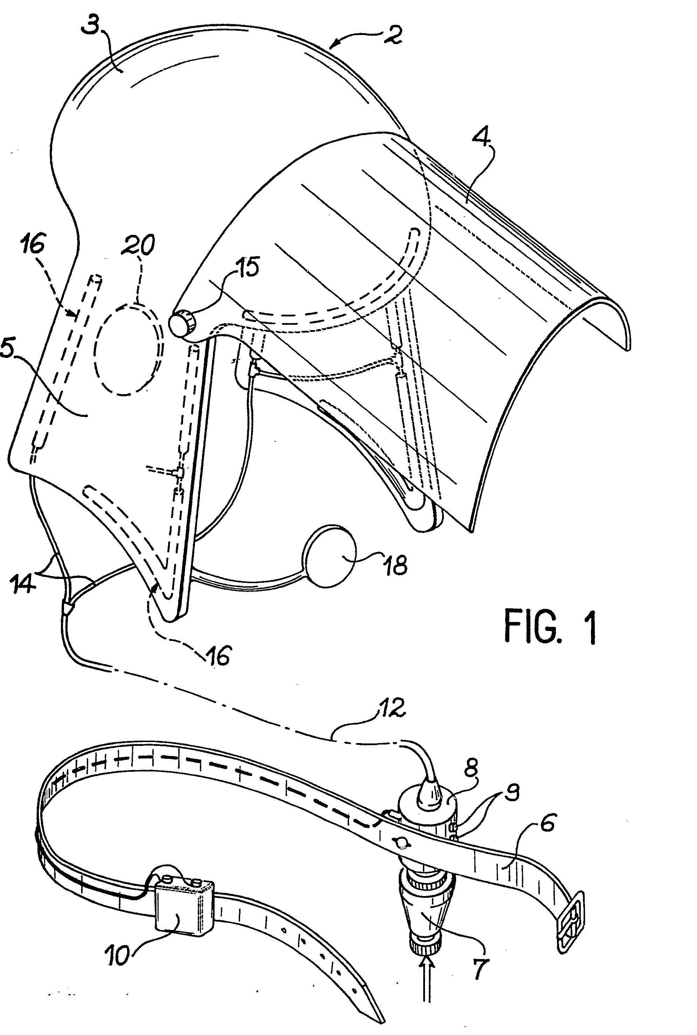

La figure 1 montre l'ensemble du heaume ergonomique objet de l'invention qui se compose essentiellement d'une partie en forme de casque 2 sur laquelle est montée une visière transparente 4 déplaçable entre une position abaissée ou position de travail et une position relevée ou position de repos : c'est cette dernière position qui est illustrée sur la figure 1. La partie 2 en forme de casque peut être réalisée en fibres de carbone légères ou en fibres de verre amalgamées par une colle synthétique ayant une bonne tenue au feu (classe MO ou M1). Quant à la visière 4, elle est avantageusement réalisée en verre synthétique à protection activée aux rayons ultraviolets alpha et bêta : elle peut être de forme semi-cylindrique, libre à sa partie inférieure et articulée à sa partie supérieure sur la partie en forme de casque, la visière 4 étant mobile en rotation autour d'un axe 15.

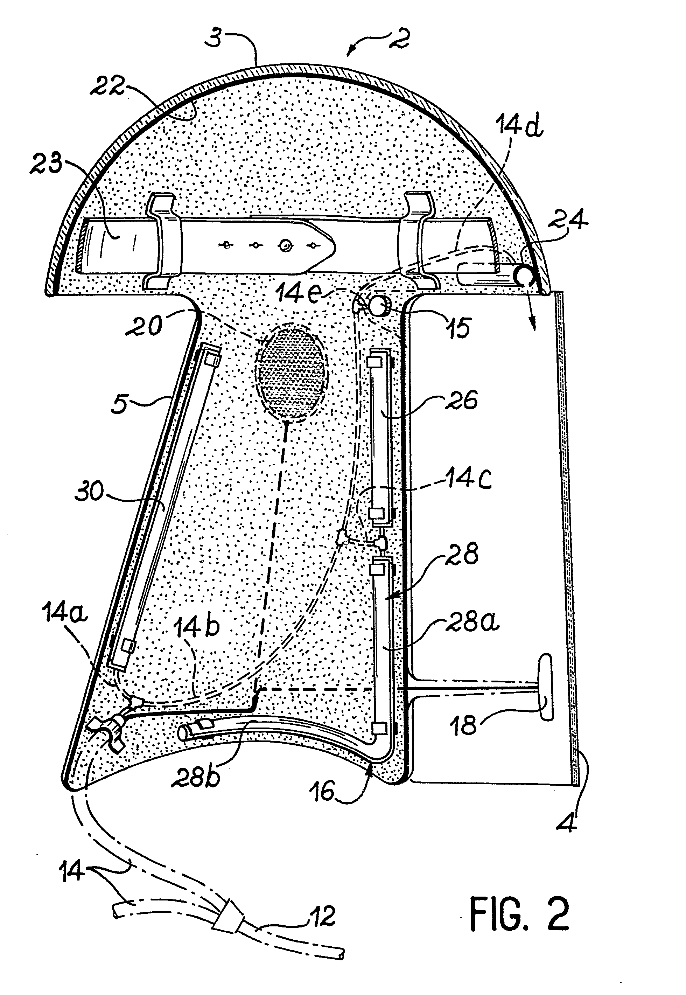

Le dispositif comporte en outre une ceinture 6, portée par l'opérateur, sur laquelle est accrochée une pompe à air 8 ; une batterie 10, également accrochée à la ceinture 6, fournit de l'énergie électrique
à la pompe. Cette dernière, qui est munie d'un filtre
tel que 7 car le heaume est destiné à être porté dans
une atmosphère polluée, est reliée au casque 2 par une
conduite souple 12 dont l'extrémité opposée à la pompe
8 est reliée à deux tuyaux souples 14 qui amènent
l'air en différents endroits du casque 2 afin d'assurer l'isolation et la ventilation du visage de l'opérateur au moyen d'un flux d'air laminaire. Le dispositif de filtration 7 peut être par exemple un filtre en
PTFE arrêtant les aérosols solides complété par une
masse de charbon actif arrêtant les vapeurs organiques. Eventuellement, on peut ajouter un préfiltre si
l'on doit travailler dans un milieu contenant des aérosols liquides. Il y a deux tuyaux souples 14, un
pour chaque côté de la partie en forme de casque du
heaume, chacun de ces tuyaux 14 se divisant encore en
plusieurs tuyaux secondaires comme cela sera explicité
plus loin en référence à la figure 2. Un certain nombre de tubes tels que 16 sont répartis autour de la
partie en forme de casque 2 pour assurer la ventilation. La pompe 8 est équipée d'un moyen de commande
qui, dans l'exemple représenté ici, se présente sous
la forme de deux boutons poussoirs 9, mais peut se
présenter sous n'importe quelle autre forme : bouton
unique, manette, etc... Le moyen de commande 9 a une
position "marche" et une position "repos". Lorsqu'on
le met en position "marche", ceci a pour effet de
faire fonctionner la pompe à débit normal et d'amener
l'air aux différents tubes 16 par l'intermédiaire de
la conduite souple 12 et des tuyaux 14 et, en outre,
d'abaisser la visière 4 en position de travail et de
la maintenir dans cette position abaissée grâce à des
moyens qui seront décrits dans la suite du présent

texte. Lorsqu'on le met en position "repos", ceci a pour effet de faire fonctionner la pompe à débit réduit et, également, de relever la visière 4 en position de repos et de la maintenir à cette position. Comme cela sera décrit en détail ci-dessous, les tubes 16 sont disposés de manière à ce que l'air de ventilation s'écoule sous la forme de plusieurs rideaux d'air formant écran autour du visage de l'opérateur aussi bien en ventilation normale qu'en ventilation réduite. Ainsi, lorsque la visière 4 est abaissée, l'intérieur du casque est ventilé et refroidi. Lorsque la visière est relevée, la ventilation réduite continue à former un écran autour du visage de l'opérateur, ce qui le protège de l'atmosphère ambiante lorsqu'il ne travaille pas. Pendant les périodes de repos, les voies respiratoires sont protégées des polluants extérieurs (poussières, gouttelettes, gaz toxiques, ...) grâce aux rideaux d'air qui s'écoulent devant le nez et la bouche et vers le torse, ce qui évite une éventuelle remontée des agents polluants.

Comme on le voit sur la figure 1, le heaume peut être équipé d'un dispositif de radio se composant d'un micro-émetteur 18 et de deux écouteurs 20, ces derniers se trouvant à l'intérieur de la partie en forme de casque au niveau des oreilles de l'utilisateur. On peut avantageusement utiliser pour cet usage le dispositif de liaison hertzienne multiplexée décrit dans le brevet français 2 430 143 appartenant au déposant : ce dispositif permet des conversations entre chaque opérateur et un poste de commandement, ainsi que des conversations entre les différents opérateurs.

La figure 2 est une vue en coupe à travers la partie en forme de casque 2 de ce heaume où l'on voit que les parties 3 recouvrant la tête et 5 protégeant les deux côtés de la tête de l'opérateur sont

**0147313**

recouvertes intérieurement d'une couche de liège 22 qui assure l'isolation phonique de l'opérateur et évite la condensation humide intérieure. De plus, cette couche de liège constitue un écran thermique complémentaire. Une courroie 23 permet l'adaptation du heaume à la taille du crâne de l'opérateur. On voit également que les écouteurs 20 de l'appareil micro-émetteur-récepteur de radio sont situés sous la masse de liège.

On voit encore sur la figure 2 l'extrémité de la conduite souple 12 qui débouche à la partie inférieure du casque 2 et est reliée à plusieurs tuyaux souples qui amènent l'air à différents orifices de sortie. Ces derniers sont constitués par des tubes fendus répartis en différents endroits du casque 2, afin de créer un rideau d'air laminaire autour du visage de l'opérateur. Dans l'exemple de réalisation représenté ici, les tubes sont disposés de la manière suivante : un tube 24 au-dessus du front de l'utilisateur assure l'écoulement de l'air vers le bas et un tube vertical 26 de chaque côté du visage dirige l'air vers l'avant de celui-ci ; deux tubes 28 se composant chacun d'une partie verticale 28a jouant le même rôle que les tubes 26 et d'une partie inférieure 28b disposée de manière à faire écouler l'air verticalement sur les épaules de l'utilisateur sont prévus sur chaque côté de la partie en forme de casque tandis que deux tubes 30 créent un rideau d'air à l'arrière du visage de l'opérateur. Le tuyau 14 alimentant la partie du casque 2 représentée à la figure 2 se divise en un tuyau 14a alimentant le tube 30 et en un tuyau 14b alimentant les autres tubes. Le tuyau 14b est relié aux tubes 26 et 28 par le tuyau 14c, au tube 24 par le tuyau 14d et à l'articulation 15 par le tuyau 14e. Cette articulation sera décrite plus loin en référence

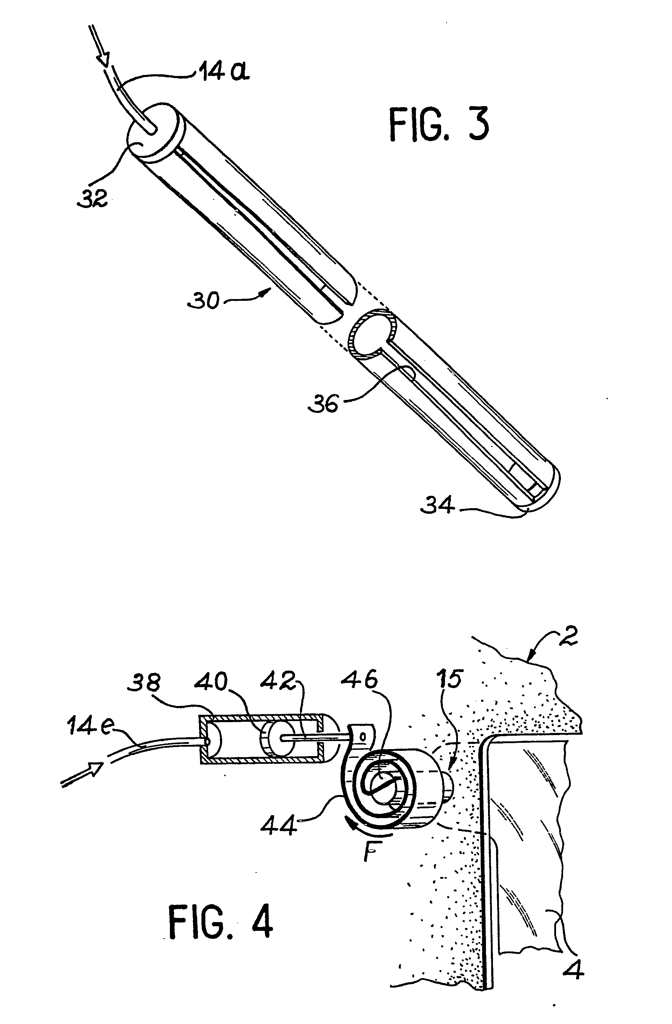

à la figure 4.

La forme des tubes apparaît mieux sur la vue en perspective de la figure 3 où l'on voit que le tube 30, par exemple, est fermé à ses deux extrémités par des capsules 32 et 34, l'air arrivant à une extrémité du tube 30 en provenance du tuyau 14a qui débouche au niveau de la capsule 32. Le tube 30 est percé d'une fente longitudinale 36 dont la largeur va en augmentant au fur et à mesure que l'on s'éloigne de l'extrémité par où débouche le tuyau 14a, c'est-à-dire en allant de la capsule 32 vers la capsule 34. Cette disposition permet d'avoir une pression constante et donc un débit constant en tout point du tube, ce qui assure un flux laminaire.

La figure 4 montre le système de relevage automatique de la visière dans le premier mode de réalisation, lorsque celle-ci est commandée par des moyens pneumatiques. Dans ce cas, l'un des tuyaux souples 14e débouche dans un cylindre 38 à l'intérieur duquel peut se déplacer un piston 40. Celui-ci est relié par une tige 42 à une extrémité d'un ressort en spirale 44 dont l'autre extrémité est fixée sur un axe 46 qui constitue l'axe de rotation 15 de la visière. Lorsque le moyen de commande 9 (figure 1) est mis en position "marche", la pompe 8 assure un débit d'air important et donc une arrivée d'air importante et une forte pression dans le cylindre 38 : ceci a pour effet de pousser le piston 40 vers la droite de la figure et donc de comprimer le ressort 44 dans le sens de la flèche F, c'est-à-dire le sens des aiguilles d'une montre vu sur la figure 4. La rotation du ressort entraîne celle de l'axe 46 et donc celle de la visière 4 ainsi que le maintien de cette dernière en position abaissée ou position de travail tant que la pompe est en fonctionnement, car celle-ci envoie en permanence

B 8069.3 JR

**0147313**

une pression d'air importante à l'intérieur du cylindre 38. Lorsque le moyen de commande 9 est mis en position "repos", la pompe 8 envoie un débit d'air réduit au casque 2, ce qui fait que la pression à l'intérieur du cylindre 38 diminue. Le piston 40 se déplace alors vers la gauche de la figure sous l'effet du ressort 44 qui se détend en se déroulant dans le sens inverse de la flèche F, ce qui a pour effet de relever la visière 4 et de la maintenir dans cette position tant que la pression d'air est faible à l'intérieur du cylindre 30.

Dans un autre mode de réalisation, on peut remplacer le système pneumatique avec le cylindre 38 et le piston 40 par un dispositif comportant un élément ou une lame réalisé en un matériau à mémorisation thermique inverse. En effet, certains matériaux, comme par exemple certains alliages de nickel et de titane, se dilatent si la température diminue et se contractent si la température augmente. Plus exactement, ces matériaux reprennent une forme qu'on leur a imposée précédemment à une certaine température lorsqu'on les porte à nouveau à cette température. On peut ainsi faire en sorte qu'un matériau se contracte au lieu de se dilater en passant d'une température donnée à une température supérieure : c'est pourquoi on parle de "mémorisation thermique inverse". On peut par exemple utiliser une lame réalisée dans un tel matériau dont une extrémité est fixée au casque 2 et l'autre à l'extrémité du ressort 44. La mise en position "marche" du moyen de commande de la pompe 8 entraîne le passage d'un courant électrique dans cet élément, donc son échauffement et sa contraction, ce qui a pour effet de contracter le ressort 44 et d'abaisser la visière 4. Si l'on met le moyen de commande en position "repos", bien que la pompe 8 continue à assurer

B 8069.3 JR

un débit d'air réduit, on interrompt le passage du courant dans l'élément en matériau à mémorisation thermique inverse : celui-ci se refroidit, s'allonge et la visière 4 se relève.

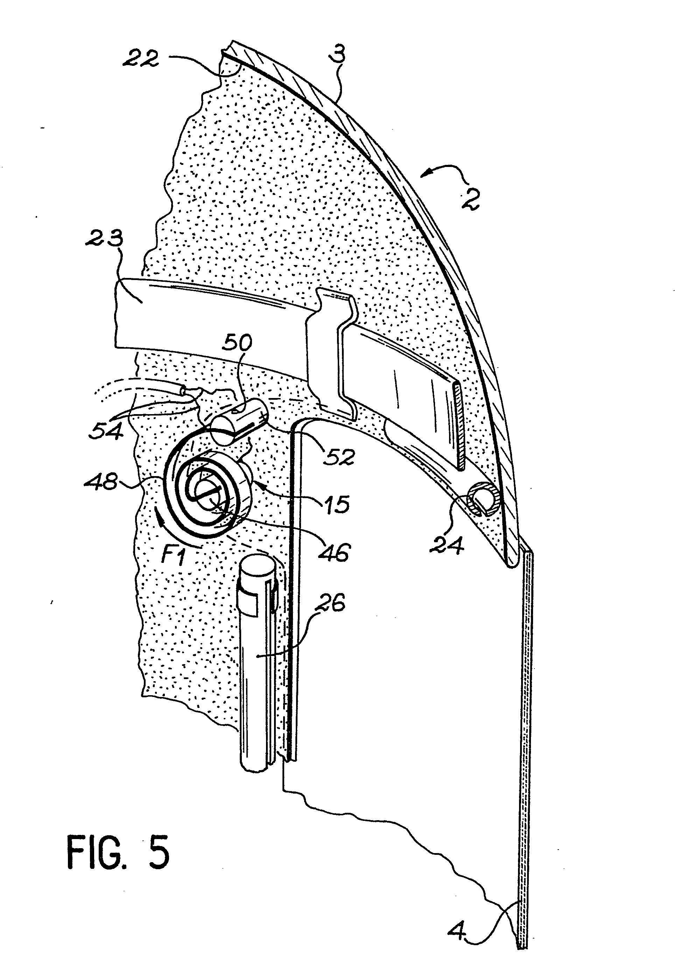

La figure 5 illustre un autre mode de réalisation dans lequel l'élément en matériau à mémorisation thermique inverse se présente sous la forme d'un ressort en spirale 48 semblable au ressort 44 de la figure 4. Une extrémité du ressort 44 est fixée à l'axe 46 qui entraîne la visière en rotation. L'autre extrémité du ressort 44 est maintenue par une goupille 50 sur un support 52 fixe par rapport à la partie en forme de casque 2. Enfin, deux fils 54 relient chaque extrémité du ressort 48 à une source de courant électrique, par exemple la batterie 10 de la figure 1.

Le fonctionnement de ce dispositif est le suivant : lorsqu'on met le moyen de commande 9 de la pompe 8 en position "marche", du courant est envoyé dans les fils 54 et donc dans le ressort 48. Celui-ci s'échauffe et donc se contracte. Comme il est fixé par une extrémité au support 52, son autre extrémité fait tourner l'axe 46 dans le sens de la flèche F1 et la visière 4 est abaissée, comme représenté à la figure 5. Si l'on met ensuite le moyen de commande 9 de la pompe 8 en position "repos", le passage du courant est interrompu dans les fils 54 et également dans le ressort 48. Celui-ci se refroidit et s'allonge : il se déroule dans le sens inverse de la flèche F1 et la rotation de l'axe 46 a pour effet de relever la visière 4.

Ainsi, l'invention propose un heaume ergonomique particulièrement intéressant, puisqu'il est facile à fabriquer en grande série et donc peu coûteux et que, d'autre part, il est léger et agréable à porter tout en assurant une protection efficace de l'uti-

lisateur. De plus, le fait d'avoir un débit d'air réduit lorsque la commande de la pompe est en position "repos" permet à l'opérateur de rester dans la zone polluée pendant les périodes d'interruption du travail tout en étant protégé de l'atmosphère environnante.

B 8069.3 JR

REVENDICATIONS

1. Heaume ergonomique, du type comportant une partie en forme de casque (2) équipée d'une visière (4) déplaçable entre une position abaissée et une position relevée ainsi qu'un système d'adduction d'air à ladite partie en forme de casque (2) au moyen d'une source d'air extérieure (8) équipée d'un moyen de commande (9), caractérisé en ce que ledit moyen de commande (9) assure simultanément la ventilation et la manoeuvre de la visière (4), ledit moyen de commande (9) ayant une position "marche" assurant simultanément l'abaissement de la visière (4) et son maintien en position abaissée ainsi que la ventilation normale et une position "repos" assurant simultanément le relevage de la visière (4) et son maintien en position relevée ainsi qu'une arrivée d'air réduite formant écran autour du visage de l'opérateur.

2. Heaume ergonomique selon la revendication 1, caractérisé en ce qu'il comprend :
- une pompe (8) servant de source d'air extérieure, cette pompe étant équipée d'une commande ayant une position "marche" assurant la ventilation normale et une position "repos" assurant une arrivée d'air réduite, cette commande assurant en outre la manoeuvre de la visière (4),
- un système de filtration des polluants équipant ladite pompe (8),
- une conduite souple (12) reliant cette pompe (8) à ladite partie en forme de casque (2), et
- plusieurs tuyaux souples (14) reliant l'extrémité de cette conduite souple (12) opposée à la pompe (8) à différents orifices de sortie.

3. Heaume ergonomique selon la revendication 2, caractérisé en ce que l'un au moins desdits

0147313

tuyaux souples (14a) débouche à une extrémité d'un tube (30) présentant une fente longitudinale (36) dont la largeur augmente au fur et à mesure qu'on s'éloigne de l'extrémité où débouche ledit tuyau souple (14a), assurant ainsi un flux d'air laminaire.

4. Heaume ergonomique selon l'une quelconque des revendications 2 et 3, caractérisé en ce que l'un au moins desdits tuyaux souples (14e) débouche dans un cylindre (38) contenant un piston mobile (40) relié à une extrémité d'un ressort en spirale (44) coopérant avec ladite visière (4).

5. Heaume ergonomique selon l'une quelconque des revendications 2 et 3, caractérisé en ce qu'il comporte :
- une source de courant électrique (10), et
- un élément (48) en matériau à mémorisation thermique inverse, ledit élément (48) étant relié électriquement à la source de courant (10) et coopérant avec ladite visière (4).

6. Heaume ergonomique selon la revendication 5, caractérisé en ce que ledit élément en matériau à mémorisation thermique inverse est un ressort en spirale (48) dont une extrémité est fixée à la partie en forme de casque (2) et dont l'autre extrémité est fixée à un axe (15) mobile en rotation par rapport à la partie en forme de casque (2) et solidaire de la visière (4) au cours de cette rotation.

7. Heaume ergonomique selon l'une quelconque des revendications 1 à 6, caractérisé en ce que ladite partie en forme de casque (2) est tapissée intérieurement d'une couche de liège (22).

8. Heaume ergonomique selon l'une quelconque des revendications 1 à 7, caractérisé en ce qu'il comporte un micro-émetteur-récepteur de radio incorporé dans la partie en forme de casque (2).

B 8069.3 JR

FIG. 1

FIG. 2

14 a

FIG. 3

32

30

36

34

38    40    42    46    15    2

14 e

44

F

4

FIG. 4

0147313

FIG. 5